# EUROPÄISCHE PATENTSCHRIFT

(11) **EP 0 741 122 B1**
(45) Veröffentlichungstag und Bekanntmachung des Hinweises auf die Patenterteilung: **25.08.1999**
(21) Anmeldenummer: 96106480.5
(22) Anmeldetag: 25.04.1996
(51) Int. Cl.: C07C 51/38, C07C 63/70, C07C 65/03, C07C 65/21, C07C 17/363, C07C 25/02

(54) **Verfahren zur Herstellung aromatischer Verbindungen durch Decarboxylierung aromatischer Carbonsäuren**
Process for preparing aromatic compounds through decarboxylation of aromatic carboxylic acids
Procédé de préparation de composés aromatiques par décarboxylation d'acides carboxyliques aromatiques

(30) Priorität: 02.05.1995 DE 19515985
(43) Veröffentlichungstag der Anmeldung: 06.11.1996
(73) Patentinhaber: Clariant GmbH, 65929 Frankfurt am Main (DE)
(72) Erfinder: Pfirmann, Ralf, Dr., 64347 Griesheim (DE); Schubert, Hans, Dr., 65779 Kelkheim (DE)

(56) Entgegenhaltungen:
- EP-A- 0 635 466
- GB-A- 818 434

## Beschreibung

Die vorliegende Erfindung betrifft ein gegenüber dem Stand der Technik verbessertes Verfahren zur Herstellung von aromatischen Verbindungen, insbesondere von aromatischen Carbonsäuren durch Decarboxylierung entsprechender aromatischer Carbonsäuren, insbesondere Phthalsäuren.

Aromatische Verbindungen und aromatische Carbonsäuren, insbesondere fluorierte aromatische Carbonsäuren stellen wertvolle Zwischenprodukte zur Herstellung antibakterieller Mittel dar (DE-OS 33 18 145, EP 0 424 850, EP 0 271 275), sie können aber auch zur Herstellung von Flüssigkristall-Materialien eingesetzt werden (EP 0 602 596).

Wie am Beispiel der Herstellung von 2,3,4,5-Tetrafluorbenzoesäure durch Decarboxylierung von Tetrafluorphthalsäure gezeigt werden kann, hat es in der Vergangenheit nicht an Versuchen gefehlt, fluorierte aromatische Carbonsäuren durch Decarboxylierung entsprechender Phthalsäuren herzustellen.

Die DE-OS 38 10 093 beschreibt die Herstellung von Tetrafluorbenzoylchlorid durch Decarboxylierung von Tetrafluorphthalsäure, die in einem Überschuß von Chinolin gelöst wird. Nach Abschluß der Umsetzung erhält man ein Chinolinsalz der Tetrafluorbenzoesäure, das anschließend in Thionylchlorid suspendiert und unter Erwärmen umgesetzt wird. Das 2,3,4,5-Tetrafluorbenzoylchlorid wird anschließend durch fraktionierte Destillation gewonnen (vgl. auch Beispiel 4).

Die EP 0 218 111 beschreibt ein Verfahren zur Herstellung von 2,3,4,5-Tetrafluorbenzoesäure, wobei Tetrafluorphthalsäure in einem polaren, aprotischen Lösungsmittel gelöst und unter Verwendung eines organischen Amins als Katalysator decarboxyliert wird. Wie aus den Beispielen 1 und 2 unter Verwendung von Dimethylsulfoxid als polares, aprotisches Lösungsmittel und Triethylamin als organisches Amin hervorgeht, wird zum einen das polaraprotische Lösungsmittel in einem großen Überschuß eingesetzt und zum anderen schließt sich an die eigentliche Decarboxylierung eine komplizierte, über mehrere Stufen verlaufende Aufarbeitung an. Man kühlt zunächst unter Verwendung von Eis ab, versetzt das Reaktionsgemisch mit einer großen Menge entmineralisiertem Wasser und gibt anschließend n-Butylether und Toluol zu. Anschließend setzt man vorsichtig unter Rühren und Kühlen konzentrierte Schwefelsäure zu, trennt die Phasen und extrahiert die wäßrige Phase zweimal mit Toluol. Die organischen Phasen werden vereinigt und anschließend dreimal mit 2 %iger wäßriger Schwefelsäure extrahiert. Die organische Phase wird anschließend über wasserfreiem Natriumsulfat getrocknet, filtriert und das Filtrat wird unter reduziertem Druck eingeengt, wobei ein Feststoff (2,3,4,5-Tetrafluorbenzoesäure) gewonnen wird.

Ein zum Verfahren der EP 0 218 111 analoges Verfahren unter Verwendung tertiärer Amine ist in der JP 63 295 529 beschrieben. Man löst 3,4,5,6-Tetrafluorphthalsäure in Tributylamin und führt die Decarboxylierung in dieser Lösung bei 130°C durch. Die Aufarbeitung des dabei anfallenden Reaktionsgemisches dürfte wegen der Ähnlichkeit von Tributylamin zu Triethylamin einen ebenso großen Aufwand, wie in der EP 0 218 111 zuvor beschrieben, erforderlich machen.

Die EP 0 194 671 betrifft ein Verfahren zur Herstellung von 2,3,4,5-Tetrafluorbenzoesäure durch Decarboxylierung von 3,4,5,6-Tetrafluorphthalsäure in einem auf einen pH-Bereich von 0,7 bis 2,2 eingestellten wäßrigen Medium. Es wird ausdrücklich auf Seite 3, Zeile 29 bis 33 darauf hingewiesen, daß die Selektivität der Decarboxylierung in Richtung 2,3,4,5-Tetrafluorbenzoesäure unzureichend ist, falls der pH-Wert des wäßrigen Mediums von dem vorstehend genannten Bereich abweicht. Die Umsetzung erfordert, wie aus den Beispielen zu entnehmen ist, vergleichsweise hohe Temperaturen von 155 bis 170°C. Die Verwendung einer relativ großen Katalysatormenge, nämlich von 0,3 Mol (NH₄)₂SO₄ und 0,8 Mol Chinolin je Mol Tetrafluorphthalsäure führt bei einer Reaktionstemperatur von 160°C und einer Reaktionszeit von 18 Stunden zu einer Ausbeute von 88,8 % 2,3,4,5-Tetrafluorbenzoesäure (vgl. Beispiel 12 in Tabelle 1).

Nachteil des Verfahrens sind zum einen die vergleichsweise hohen Reaktionstemperaturen und zum anderen die relativ langen Reaktionszeiten. Darüberhinaus ergeben sich aufgrund des niedrigen pH-Wertes der wäßrigen Lösung, der durch die Konzentration der im Wasser gelösten 3,4,5,6-Tetrafluorphthalsäure festgelegt wird, erhebliche Probleme hinsichtlich Korrosion, verursacht durch die Umsetzung derartiger wäßriger korrosiver Lösungen bei hohen Temperaturen.

Im Hinblick auf die Nachteile der vorstehend beschriebenen Verfahren des Standes der Technik stellt sich die Aufgabe, ein Verfahren zu entwickeln, das zum einen sich nicht nur auf die Herstellung von 2,3,4,5-Tetrafluorbenzoesäure durch Decarboxylierung von 3,4,5,6-Tetrafluorphthalsäure beschränkt, sondern sich in größerem Umfange auch auf andere Carbonsäuren anwenden läßt, und zum anderen die Nachteile der vorstehend beschriebenen Verfahren, beispielsweise die komplizierte Aufarbeitung des anfallenden Reaktionsgemisches, die Anwendung hoher Temperaturen und langer Reaktionszeiten und den Einsatz korrosiver wäßriger Lösungen, vermeidet. Darüberhinaus soll das Verfahren sich ohne großen technischen Aufwand auf einfache Weise durchführen lassen und zudem von vergleichsweise leicht zugänglichen Ausgangsstoffen und Hilfsstoffen ausgehen.

Diese Aufgabe wird gelöst durch ein Verfahren zur Herstellung von Verbindungen der allgemeinen Formel 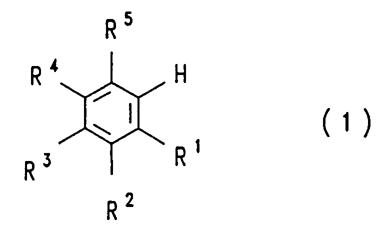 worin die Reste R¹, R², R³, R⁴ und R⁵ gleich oder verschieden sind und für COOH, H, F, Cl, Br, CF₃, OH, einen Alkoxy- oder Alkylrest mit jeweils 1 bis 4 Kohlenstoffatomen oder einen Rest -NR⁶R⁷ stehen, in welchem R⁶ and R⁷ gleich oder verschieden sind und für H, einen Alkylrest mit 1 bis 4 Kohlenstoffatomen oder einen Phenylrest stehen. Es ist dadurch gekennzeichnet, daß man eine Verbindung der allgemeinen Formel 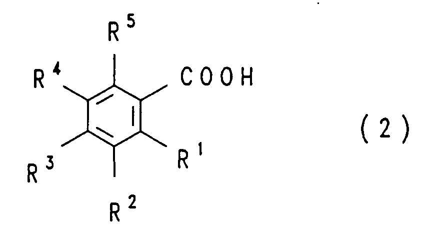 worin die Reste R¹, R², R³, R⁴ und R⁵ gleich oder verschieden sind und die vorstehend genannte Bedeutung haben oder abweichend hiervon anstelle von H für COOH stehen können, in Wasser löst, die wäßrige Lösung mit einem in Wasser nichtlöslichen, unter den Reaktionsbedingungen inerten Amin versetzt und die Decarboxylierung in Anwesenheit oder Abwesenheit eines in Wasser nichtlöslichen, unter den Reaktionsbedingungen inerten Lösungsmittels und in Anwesenheit oder Abwesenheit eines Decarboxylierungskatalysators bei einem pH-Wert von 3 bis 9 und einer Temperatur von 70 bis 210°C durchführt.

Das erfindungsgemäße Verfahren ist nicht auf die Decarboxylierung einer einzigen COOH-Gruppe beschränkt. Es läßt sich auch auf die Decarboxylierung zweier oder mehrerer COOH-Gruppen anwenden, wobei in diesen Fällen die Reste R¹, R², R³,R⁴ und R⁵ gleich oder verschieden sind und, abweichend von der in der Verbindung der Formel (1) genannten Bedeutung, anstelle von H für COOH stehen. Durch die Abspaltung von CO₂ bildet sich aus der COOH-Gruppe des Einsatzstoffes der Formel (2) ein H im Endprodukt der Formel (1). Dadurch kann es zu der von der ursprünglich in Formel (1) genannten Bedeutung abweichenden Bedeutung der Reste R¹ bis R⁵ im Einsatzstoff der Formel (2) kommen.

Das erfindungsgemäße Verfahren besitzt gegenüber den Verfahren des Standes der Technik mehrere Vorteile. Zum einen erweist sich die Aufarbeitung des anfallenden Reaktionsgemisches als unproblematisch, da lediglich die organische Phase von der das Wertprodukt enthaltenden wäßrigen Phase abzutrennen ist. Somit entfällt eine komplizierte Abtrennung des Wertproduktes, beispielsweise 2,3,4,5-Tetrafluorbenzoesäure, aus einem als Lösungsmittel verwendeten Amin oder aus einem ein Amin und ein Lösungsmittel enthaltenden Gemisch. Das durch einfache Phasentrennung erhaltene Amin oder das ein Amin und ein Lösungsmittel enthaltende Gemisch kann direkt oder gegebenenfalls nach Reinigung wieder in die Decarboxylierungsreaktion eingesetzt werden.

Das erfindungsgemäße Verfahren läßt sich in einer Reihe von Fällen, beispielsweise bei der Herstellung von 2,3,4,5-Tetrafluorbenzoesäure, bei vergleichsweise niedrigen Temperaturen (siehe auch Beispiel 2) unter relativ kurzen Reaktionszeiten durchführen. Darüberhinaus gestattet das erfindungsgemäße Verfahren, in einem pH-Bereich zu arbeiten, wo Korrosionsprobleme entweder nur eine untergeordnete Rolle spielen oder gar nicht mehr vorhanden sind.
Ein weiterer Vorteil des erfindungsgemäßen Verfahrens besteht darin, daß man nicht auf den Einsatz reiner Ausgangsstoffe angewiesen ist, sondern rohe Ausgangsstoffe, wie sie beispielsweise in deren Herstellung mitunter anfallen, verwenden kann. Derartige Ausgangsstoffe enthalten naturgemäß im gewissen Umfange stets Nebenkomponenten, beispielsweise in Wasser lösliche Salze.

Eine Verfahrensvariante besteht darin, daß man eine Verbindung der Formel (2) einsetzt, worin R¹, R², R³, R⁴ und R⁵ gleich oder verschieden sind und für COOH, H, F, Cl, OH oder für einen Alkoxyrest von 1 bis 4 Kohlenstoffatomen stehen, insbesondere gleich oder verschieden sind und für COOH, H, F, OH oder einen Alkoxyrest mit 1 bis 4 Kohlenstoffatomen stehen. Von besonderem Interesse sind Verbindungen der Formel (2), worin 0 bis 2 der Reste R¹ bis R⁵ für COOH stehen, aber auch Verbindungen der Formel (2), worin R¹ oder R⁵ für COOH stehen.

Wie eingangs bereits erwähnt, betrifft das erfindungsgemäße Verfahren auch die Herstellung fluorierter Verbindungen. Hierbei setzt man üblicherweise eine Verbindung der Formel (2) ein, worin ein bis vier der Reste R¹ bis R⁵, insbesondere zwei bis vier der Reste R¹ bis R⁵, bevorzugt zwei oder drei der Reste R¹ bis R⁵ für F stehen.
In einer Reihe von Fällen setzt man eine Verbindung der Formel (2) ein, worin einer der Reste R¹ bis R⁵ für OH oder eine Alkoxygruppe mit 1 bis 4 Kohlenstoffatomen, insbesondere für OH, steht.

Man kann in die Umsetzung eine wäßrige Lösung einsetzen, die das Ausgangsprodukt in relativ niedriger Konzentration oder auch in relativ hoher Konzentration enthält. Üblicherweise setzt man eine wäßrige Lösung ein, die 1 bis 50, insbesondere 10 bis 30, bevorzugt 15 bis 25 Gew.-% der Verbindung der Formel (2) und gegebenenfalls weitere wasserlösliche Salze oder wasserlösliche Verbindungen, beispielsweise in einer Menge von 0,1 bis 60, insbesondere 20 bis 40 Gew.-% enthält.

Als Beispiele für Verbindungen der Formel (2) seien, ohne Anspruch auf Vollständigkeit zu erheben, genannt: Benzoesäuren, Phthalsäuren, Isophthalsäuren, Terephthalsäuren, die jeweils gegebenenfalls chloriert, bromiert oder fluoriert sind, beispielsweise 2,3,5-Trifluorbenzoesäure, 2,3,4,5-Tetrafluorbenzoesäure, 2,3,4,5-Tetrachlorbenzoesäure, 3,5-Dichlorphthalsäure, 3,6-Dichlorphthalsäure, 4,5-Dichlorphthalsäure, 3,5-Difluorphthalsäure, 3,6-Difluorphthalsäure, 4,5-Difluorphthalsäure, 3,4,5-Trifluorphthalsäure, 3,4,6-Trifluorphthalsäure, 2,4,5-Trifluorisophthalsäure, Difluor- und Trifluorphthalsäuren und -isophthalsäuren, in denen F gegen OH, OCH₃, OC₂H₅, CF₃ oder NH₂ ausgetauscht sein kann, beispielsweise 4-Hydroxy-3,5,6-trifluorphthalsäure, 4-Methoxy-3,5,6-trifluorphthalsäure, 4-Ethoxy-3,5,6-trifluorphthalsäure, 4-Trifluormethyl-3,5,6-trifluorphthalsäure, 4-Amino-3,5,6-trifluorphthalsäure und 4-Dimethylamino-3,5,6-trifluorphthalsäure, ferner Trifluorterephthalsäuren, Trichlorterephthalsäuren, Tribromterephthalsäuren, 2,5-Dichlorterephthalsäure, 2,5-Difluorterephthalsäure, 2,5-Dibromterephthalsäure, 3,5-Dichlorterephthalsäure, 3,5-Difluorterephthalsäure, 3,5-Dibromterephthalsäure und tetrachlorierte Phthalsäuren, wie Tetrafluorphthalsäure, Tetrachlorphthalsäure, Tetrabromphthalsäure, Tetrafluorisophthalsäure, Tetrafluorterephthalsäure, Tetrachlorterephthalsäure und Tetrabromterephthalsäure.
Es lassen sich auch Verbindungen der vorstehend genannten Art einsetzen, in denen F gegen OH, OCH₃, OC₂H₅, CF₃ oder NH₂ ausgetauscht sein kann.

Das in Wasser nichtlösliche Amin kann in vergleichsweise geringen Mengen, aber auch in relativ hohen Mengen Anwendung finden. Üblicherweise setzt man je Mol der Verbindung der Formel (2) 0,001 bis 50, insbesondere 0,01 bis 2, bevorzugt 0,05 bis 1, besonders bevorzugt 0,1 bis 0,5 Mol des in Wasser nichtlöslichen Amins ein.

Unter dem Begriff in Wasser nichtlösliches Amin werden solche Amine verstanden, die sich entweder in Wasser nur in geringem Umfange oder gar nicht lösen. Üblicherweise verwendet man als in Wasser nichtlösliches Amin ein Alkylamin mit 6 bis 30 Kohlenstoffatomen, ein Dialkylamin mit 6 bis 30 Kohlenstoffatomen je Alkylrest, ein Trialkylamin mit 4 bis 30 Kohlenstoffatomen je Alkylrest, eine N-enthaltende heterocyclische Verbindung oder ein Gemisch der vorstehend genannten Stoffe, insbesondere ein Alkylamin mit 8 bis 20 Kohlenstoffatomen im Alkylrest, ein Dialkylamin mit 8 bis 20 Kohlenstoffatomen je Alkylrest, ein Trialkylamin mit 6 bis 20 Kohlenstoffatomen je Alkylrest, ein gegebenenfalls alkyliertes Chinolin oder Pyridin, beispielsweise Collidin, Lutidin oder Picolin oder ein Gemisch der vorstehend genannten Stoffe, bevorzugt ein Trialkylamin mit 6 bis 20, insbesondere 6 bis 14, bevorzugt 8 bis 12 Kohlenstoffatomen je Alkylrest oder ein Gemisch dieser Trialkylamine.

Ohne Anspruch auf Vollständigkeit zu erheben, seien als Beispiele für geeignete Amine genannt: n-Hexylamin, Isohexylamin, n-Heptylamin, Isoheptylamin, n-Octylamin, Isooctylamin, n-Nonylamin, Isononylamin, n-Decylamin, Isodecylamin, n-Dodecylamin, Isododecylamin, n-Hexadecylamin, Isohexadecylamin, Di-n-hexylamin, Diisohexylamin, Di-n-heptylamin, Diisoheptylamin, Di-n-octylamin, Diisooctylamin, Di-n-nonylamin, Diisononylamin, Di-n-decylamin, Diisodecylamin, Di-n-dodecylamin, Diisododecylamin, Di-n-hexadecylamin, Diisohexadecylamin, Tri-n-hexylamin, Tri-isohexylamin, Tri-n-heptylamin, Triisoheptylamin, Tri-n-octylamin, Triisoctylamin, Tri-n-decylamin, Triisodecylamin, Tri-n-dodecylamin, Triisododecylamin, Trialkylamine mit geradkettigen und/oder verzweigten Ketten mit 6 bis 14 Kohlenstoffatomen, Pyridin, α-Picolin, β-Picolin, γ-Picolin, 2,4-Dimethylpyridin(α,γ-Lutidin), 2,6-Di-tert.-butylpyridin, 2,4,6-Trimethylpyridin (Collidin), Triethylpyridin, Chinolin, Methylchinoline, Ethylchinoline, gemischte Amine wie Butyldihexylamin, Dioctyldecylamin, Hexyldioctylamin, Dihexyloctylamin, Diheptyloctylamin, Didecyloctylamin, Didodecyloctylamin, Didodecyldecylamin, Didecyldodecylamin, Dioctyldodecylamin, Dinonyloctylamin, Dinonyldecylamin, Dinonyldodecylamin.

Es lassen sich generell auch beliebige Mischungen der vorstehend genannten, in Wasser nicht löslichen Amine, insbesondere Gemische verschiedener Alkyl-, Dialkyl- und Trialkylamine, vorzugsweise Gemische verschiedener Trialkylamine mit 6 bis 14, insbesondere 8 bis 12 Kohlenstoffatomen einsetzen.

Man kann die Reaktion in Anwesenheit oder Abwesenheit eines in Wasser nichtlöslichen, unter den Reaktionsbedingungen inerten Lösungsmittels durchführen. Üblicherweise setzt man das inerte Lösungsmittel in einer Menge von 1 bis 200, insbesondere 2 bis 50, bevorzugt 5 bis 20 Volumen-%, bezogen auf die wäßrige Lösung, ein.

Das inerte Lösungsmittel soll zum einen in Wasser nicht löslich sein, zum anderen nach Ende der Reaktion eine gute Phasentrennung ermöglichen und ferner das in Wasser nichtlösliche Amin lösen.

Geeignet als inertes Lösungsmittel sind halogenierte oder nicht halogenierte aliphatische Kohlenwasserstoffe, halogenierte oder nicht halogenierte aromatische Kohlenwasserstoffe oder Ether, insbesondere chlorierte oder nicht chlorierte aromatische Kohlenwasserstoffe, bevorzugt chlorierte oder nicht chlorierte Benzole. Ohne Anspruch auf Vollständigkeit zu erheben, seien als Beispiele für inerte Lösungsmittel Toluol, o-Xylol, m-Xylol, p-Xylol, Gemische isomerer Xylole, Diphenylether, Diphenylmethan, Diphenyl, o-Chlortoluol, m-Chlortoluol, p-Chlortoluol, o-Dichlorbenzol, m-Dichlorbenzol, p-Dichlorbenzol genannt.
Es lassen sich jedoch auch beliebige Mischungen der vorstehend genannten Lösungsmittel als inertes Lösungsmittel verwenden.

Wie eingangs erwähnt, läßt sich die Umsetzung in Anwesenheit oder Abwesenheit eines üblichen Decarboxylierungskatalysators durchführen. Geeignet als Decarboxylierungskatalysator ist Kupfer, eine Kupfer(I)verbindung oder eine Kupfer(II)verbindung, beispielsweise Kupfer(I)oxid, Kupfer(II)oxid, Kupfer(I)sulfat, Kupfer(II)sulfat, Kupfer(I)chlorid, Kupfer(II)chlorid, Kupfer(I)fluorid, Kupfer(II)fluorid, Kupfercarbonat, Kupfer(I)hydroxid, Kupfer(II)hydroxid, bevorzugt Kupfer(I)oxid und Kupfer(II)oxid. Es lassen sich auch beliebige Mischungen der vorstehend genannten Stoffe verwenden.

Üblicherweise setzt man den Decarboxylierungskatalysator in einer Menge von 0,1 bis 10, insbesondere 0,5 bis 3 Mol-%, bezogen auf die Verbindung der Formel (2), ein.

Wie bereits erwähnt, läßt sich die Decarboxylierung in einem relativ weiten pH-Bereich, nämlich von pH 3 bis pH 9 durchführen.

In einer Reihe von Fällen läßt sich die Decarboxylierung bei einem pH-Wert von 3,5 bis 8, insbesondere bei einem pH-Wert von 4 bis 7, bevorzugt 5 bis 7, mit gutem Erfolg durchführen.

In vielen Fällen hat es sich als ausreichend erwiesen, die Decarboxylierung bei einer Temperatur von 80 bis 180, insbesondere 90 bis 160°C durchzuführen.

Die für die Decarboxylierung anzuwendende Reaktionstemperatur hängt aber auch in gewissem Umfange von der Art der jeweiligen Decarboxylierung ab. Setzt man eine Verbindung der Formel (2), die insgesamt zwei oder mehr COOH-Reste enthält ein, und spaltet lediglich eine COOH-Gruppe ab, läßt sich diese Decarboxylierung in Abwesenheit des Decarboxylierungskatalysators bei vergleichsweise niedrigen Temperaturen durchführen. Beabsichtigt man hingegen aus der vorstehend genannten Verbindung der Formel (2) zwei oder mehr COOH-Gruppen abzuspalten, so empfiehlt es sich, die Umsetzung entweder von Anbeginn an bei vergleichsweise hohen Temperaturen beispielsweise bei wenigstens 120°C, insbesondere wenigstens 130°C, bevorzugt wenigstens 140°C und vorzugsweise in Gegenwart des Decarboxylierungskatalysators durchzuführen, oder aber die erste der COOH-Gruppen bei relativ niedriger Temperatur und in Abwesenheit des Decarboxylierungskatalysators abzuspalten und die weiteren COOH-Gruppen anschließend wie vorstehend erläutert, bei höheren Temperaturen und vorzugsweise in Anwesenheit des Decarboxylierungskatalysators abzuspalten.

Das vorliegende Verfahren umfaßt insbesondere auch folgende Variante, die sich dadurch auszeichnet, daß man die Decarboxylierung einer Verbindung der Formel (2), worin ein oder zwei der Reste R¹ bis R⁵ oder der Rest R¹ oder der Rest R⁵ für COOH stehen, zu einer Verbindung der Formel (1), worin ein oder zwei der Reste R¹ bis R⁵ oder der Rest R¹ oder der Rest R⁵ für COOH stehen, bei einer Temperatur von 80 bis 130, insbesondere 85 bis 125, bevorzugt 90 bis 120°C durchführt. Diese Variante trifft insbesondere für den Fall zu, daß aus der Verbindung der Formel (2), die insgesamt zwei oder mehr COOH-Gruppen enthält, lediglich eine COOH-Gruppe abgespalten werden soll.

Das Verfahren läßt sich bei Unterdruck, Normaldruck oder Überdruck ausführen.

Die nachfolgenden Beispiele beschreiben die Erfindung näher, ohne sie darauf zu beschränken.

### Experimenteller Teil

### Beispiel 1

### Herstellung von 3-Hydroxy-2,4,5-trifluorbenzoesäure

450,3 g einer stark alkalischen, wäßrigen Lösung die 41,8 g (0,177 Mol) 4-Hydroxy-3,5,6-trifluorphthalsäure in Form entsprechender Alkalisalze enthält, werden mit 12,6 g eines Gemisches verschiedener aliphatischer Trialkylamine mit jeweils 6 bis 14 Kohlenstoffatomen im Alkylrest (Hostarex A 327; ein Handelsprodukt der Hoechst AG) versetzt und mit ingesamt 166,8 g einer 30 %igen wäßrigen Salzsäure auf pH 5 eingestellt und anschließend über einen Zeitraum von 6 Stunden bei 105°C unter Rühren erwärmt. Der pH-Wert verändert sich infolge der Decarboxylierung und wird nach einer Stunde durch Zugabe von 23,4 g 30 %iger wäßriger Salzsäure und nach 3 Stunden durch Zugabe von 15,9 g 30 %iger wäßriger Salzsäure entsprechend korrigiert (auf pH 5 eingestellt).
Anschließend wird abgekühlt und zur weiteren Verarbeitung wird ein pH-Wert von 8 eingestellt. Die Wasserphase (601 g) enthält 31,3 g (91,3 % d.Th.) 3-Hydroxy-2,4,5-trifluorbenzoesäure (bestimmt durch kalibrierte HPLC-Chromatographie).
Beabsichtigt man die 3-Hydroxy-2,4,5-trifluorbenzoesäure zu isolieren, so trennt man die Phasen nach der Decarboxylierung, stellt die wäßrige Phase auf einen pH-Wert von 1 bis 2 ein und extrahiert kontinuierlich, beispielsweise mit Methyl-tert.-butylether oder Butylacetat. Aus der organischen Phase gewinnt man nach Trocknen und Filtrieren und Entfernen des Lösungsmittels einen festen Rückstand, aus dem durch Umkristallisieren reine 3-Hydroxy-2,4,5-trifluorbenzoesäure gewonnen wird.

### Beispiel 1a

### Herstellung von 3-Hydroxy-2,4,5-trifluorbenzoesäure

Man arbeitet wie in Beispiel 1 beschrieben, setzt jedoch lediglich 9 g eines Gemisches verschiedener aliphatischer Trialkylamine mit jeweils 6 bis 14 Kohlenstoffatomen im Alkylrest (Hostarex A 327; ein Handelsprodukt der Hoechst AG) und 518 g einer wäßrigen Lösung, die 29,8 g (0,126 Mol) 4-Hydroxy-3,5,6-trifluorphthalsäure in Form entsprechender Alkalisalze enthält, ein und erhält unter Zugabe von insgesamt 158,3 g 30 %iger wäßriger Salzsäure in ebenfalls 6 Stunden 23,25 g (95,9 % d.Th.) 3-Hydroxy-2,4,5-trifluorbenzoesäure.

Verfährt man wie angegeben, setzt jedoch 30 ml Xylol oder 50 ml Diphenylmethan zu Beginn der Umsetzung oder nach Abschluß der Umsetzung zu, so erhält im wesentlichen dieselben Ergebnisse.

Man kann die Umsetzung statt bei 105°C auch bei einer Temperatur ab 85°C durchführen, muß dabei allerdings eine Verlängerung der Reaktionszeit in Kauf nehmen. Eine Umsetzung bei 90°C erfordert beispielsweise eine Reaktionszeit von 24 Stunden, eine Umsetzung bei 95 bis 100°C lediglich eine Reaktionszeit von 9 Stunden.
Führt man die Umsetzung bei einem pH-Wert von 4 oder 8 durch, so verlängert sich die Reaktionszeit ebenfalls. Führt man jedoch wie oben beschrieben, die Umsetzung bei pH 5,5 bis 6 durch, so ist die Umsetzung bei einer Reaktionstemperatur von 100 bis 103°C bereits nach 4 bis 5 Stunden beendet.

Beabsichtigt man die 3-Hydroxy-2,4,5-trifluorbenzoesäure zu isolieren, so trennt man die Phasen nach der Decarboxylierung, stellt die wäßrige Phase auf einen pH-Wert von 1 bis 2 ein und extrahiert kontinuierlich, beispielsweise mit Methyl-tert.-butylether oder Butylacetat. Aus der organischen Phase gewinnt man nach Trocknen und Filtrieren und Entfernen des Lösungsmittels einen festen Rückstand, aus dem durch Umkristallisieren reine 3-Hydroxy-2,4,5-trifluorbenzoesäure gewonnen wird.

### Beispiel 2

### Herstellung von 2,3,4,5-Tetrafluorbenzoesäure

55,3 g einer braunen, wäßrigen Lösung aus alkalischer Hydrolyse, die 64,5 g (0,271 Mol) 3,4,5,6-Tetrafluorphthalsäure enthält, werden mit 50 g eines Wärmeträgeröls, das als Hauptbestandteile alkylsubstituierte Aromaten enthält, versetzt. Man setzt 30 g eines Gemisches von Trialkylaminen mit 6 bis 10 Kohlenstoffatomen (Hostarex A 324; ein Handelsprodukt der Hoechst AG) zu und stellt den pH-Wert durch Zugabe von 55 g 96 %iger Schwefelsäure auf pH 6 bis 7 ein. Die Mischung wird unter kräftigem Rühren über einen Zeitraum von 9 Stunden auf 110°C erhitzt. Aufgrund des hohen Salzgehaltes der wäßrigen Lösung entspricht diese Temperatur der Rückflußtemperatur. Das Fortschreiten der Umsetzung wird mittels HPLC-Chromatographie bestimmt. Man erhält eine Mischung, die 48,4 g (92 % d.Th.) 2,3,4,5-Tetrafluorbenzoesäure enthält. Die Mischung kann direkt weiterverarbeitet werden.

Zur Reindarstellung von 2,3,4,5-Tetrafluorbenzoesäure trennt man die organische Phase von der wäßrigen Phase, stellt durch Zugabe von Säure einen pH-Wert von 1 bis 2 ein und filtriert die ausgefallene 2,3,4,5-Tetrafluorbenzoesäure ab. Die weitere Reinigung kann entweder durch Umkristallisieren oder durch fraktionierte Destillation vorgenommen werden.

### Beispiel 3

### Herstellung von Methoxytrifluorbenzoesäure

274 g einer wäßrigen, alkalischen Lösung, die 25 g (0,1 Mol) 4-Methoxy-3,5,6-trifluorphthalsäure in Form ihrer Alkalisalze enthält, werden mit 10 g Trioctylamin versetzt und durch Zugabe von 62 %iger Bromwasserstoffsäure bei einer Temperatur von 107°C auf pH 7 gestellt. Man läßt die Mischung bei dieser Temperatur 14 Stunden lang unter Rühren reagieren, wobei man durch Zugabe von Bromwasserstoffsäure von Zeit zu Zeit auf den vorgegebenen Wert von pH 7 einstellt.

Das Fortschreiten der Umsetzung wird mittels HPLC-Chromatographie verfolgt. Die Reaktion ist, erkennbar auch an der Beendigung der Gasentwicklung nach Ablauf der Reaktionszeit von 14 Stunden beendet. Man kühlt das Gemisch auf eine Temperatur von 0 bis 5°C ab und setzt bis zu einem pH-Wert von 1 Schwefelsäure zu und saugt anschließend die Methoxytrifluorbenzoesäure ab. Man erhält nach Trocknen 18,9 g (85 bis 92 %) bräunliches Pulver (Reingehalt ca. 90 %), das als Rohprodukt zur weiteren Verarbeitung eingesetzt werden kann oder durch Umkristallisieren gereinigt wird.

### Beispiel 4

### Herstellung von Trifluorbenzoesäure

Man löst 22,0 g (0,1 Mol) 3,5,6-Trifluorphthalsäure in 50 g Wasser auf, setzt 5 g Tridecylamin zu und stellt unter Rühren durch Zugabe von 30 %iger wäßriger Natronlauge auf pH-Wert von 6,5 ein. Anschließend erhitzt man über einen Zeitraum von 10 Stunden auf 100°C und hält den pH-Wert durch Zugabe von Phosphorsäure auf 6,5 konstant, kühlt nach Beendigung der Umsetzung (Überwachung durch HPLC-Chromatographie) auf 5°C ab, setzt bis zu einem pH-Wert 1 Säure zu, erwärmt auf 40°C und trennt die organische Phase von der wäßrigen Phase. Die wäßrige Phase enthält 15,8 g (0,0898 Mol; 90 %) Trifluorbenzoesäure als Isomerengemisch (bestimmt durch kalibrierte HPLC-Chromatographie) und kann unmittelbar weiterverarbeitet werden.

### Beispiel 5

### Herstellung eines Gemisches von Chlortrifluor- und Dichlordifluorbenzoesäuren

Man arbeitet wie in Beispiel 4 angegeben, setzt jedoch nicht ein vollständig fluoriertes Produkt, sondern ein Gemisch aus Chlortrifluorphthalsäuren und Dichlordifluorphthalsäuren (entsprechend einer Menge von 30 g) ein, so erhält man durch Extraktion der wäßrigen Phase mit Butylacetat, Abtrennung der organischen Phase, Trocknen über MgSO₄ und Entfernen des Lösungsmittels im Vakuum 23,3 g eines Gemisches der entsprechenden Chlortrifluorbenzoesäuren und Dichlordifluorbenzoesäuren.

### Beispiel 6

### Herstellung von 1,2,3,4-Tetrafluorbenzol

Man löst 19,4 g (0,1 Mol) 2,3,4,5-Tetrafluorbenzoesäure in 40 g Wasser auf, setzt 20 g eines Gemisches von Trialkylaminen mit 6 bis 14 Kohlenstoffatomen (Hostarex A 327; ein Handelsprodukt der Hoechst AG) und 0,2 g Kupfer (I)-oxid zu und stellt den pH-Wert durch Zusatz von 30 %iger wäßriger Natronlauge auf einen pH-Wert von 7 ein. Man führt die Decarboxylierung in einem Autoklaven bei 155°C in einem Zeitraum von 4 Stunden durch, wobei das abgespaltene Kohlendioxid bei einem Druck von 12 bar über einen Druckkühler entspannt und das hierbei übergehende Destillat aufgefangen wird (Kühlfalle -78°C). Nach Abschluß der Umsetzung kühlt man ab und destilliert das 1,2,3,4-Tetrafluorbenzol bei 100°C mittels Wasserdampf ab und erhält 1,5 l Destillat, das mit dem zuvor aufgefangenen Destillat vereinigt wird. Die vereinigten Destillate extrahiert man mittels Dichlormethan, trennt die organische Phase ab, trocknet über Magnesiumsulfat, filtriert und entfernt das Lösungsmittel im Vakuum. Man erhält 10,5 g rohes, leicht gelbliches 1,2,3,4-Tetrafluorbenzol, das durch fraktionierte Destillation auf eine sehr hohe Reinheit gebracht werden kann.

### Vergleichsversuch

### Herstellung von 2,3,4,5-Tetrafluorbenzoesäure (Decarboxylierung von Tetrafluorphthalsäure in wasserfreier Aminlösung)

Man löst 11,6 g (48,7 mMol) 3,4,5,6-Tetrafluorphthalsäure in 40 g eines Gemisches von Trialkylaminen mit 6 bis 14 Kohlenstoffatomen (Hostarex A 327; ein Handelsprodukt der Hoechst AG). Man heizt die dabei erhaltene leicht gelbliche klare Lösung allmählich (10°C pro Stunde) bis auf 100°C auf. Bei 100°C kann allerdings noch keinerlei Gasentwicklung (CO₂-Abspaltung), die ein Maß für das Ablaufen der Decarboxylierung ist, beobachtet werden. Nach 2 Stunden bei 100°C erhöht man die Temperatur für 30 Minuten auf 115°C und anschließend für 30 Minuten auf 120°C. Weder bei 115°C noch bei 120°C wird eine Gasentwicklung beobachtet.

Erst bei Erreichen einer Temperatur von 125°C tritt eine leichte Gasentwicklung ein. Man läßt bei dieser Temperatur weitere 4 Stunden reagieren, führt schließlich die Umsetzung bei 140°C in einem Zeitraum von 1,5 Stunden zu Ende und erhält eine orangefarbene Lösung.

Zur weiteren Aufarbeitung wird die 2,3,4,5-Tetrafluorbenzoesäure enthaltende Aminlösung mit 150 g Wasser versetzt, mit 12 g 35 %iger wäßriger Natronlauge auf pH 13 eingestellt und anschließend 5 mal mit je 75 ml Dichlormethan extrahiert. Die verbleibende wäßrige Phase wird mit 14 g einer 30 %igen wäßrigen Salzsäure angesäuert und anschließend 4 mal mit je 50 ml Methyl-tert.-butylether extrahiert. Die wäßrige Phase wird verworfen.

Man vereinigt die Methyl-tert.-butylether-Phasen und entfernt das Lösungsmittel im Vakuum, wobei man als Rückstand 8,7 g (44,8 mMol) farblose bis leicht gelbliche 2,3,4,5-Tetrafluorbenzoesäure vom Schmelzpunkt 85,2°C in Form von Pulver und Brocken erhält.

## Patentansprüche

1. Verfahren zur Herstellung von Verbindungen der allgemeinen Formel 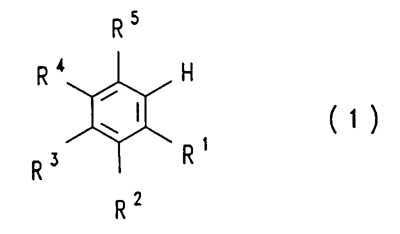 worin die Reste R¹, R², R³, R⁴ und R⁵ gleich oder verschieden sind und für COOH, H, F, Cl, Br, CF₃, OH, einen Alkoxy- oder Alkylrest mit jeweils 1 bis 4 Kohlenstoffatomen oder einen Rest -NR⁶R⁷ stehen, in welchem R⁶ and R⁷ gleich oder verschieden sind und für H, einen Alkylrest mit 1 bis 4 Kohlenstoffatomen oder einen Phenylrest stehen, dadurch gekennzeichnet, daß man eine Verbindung der allgemeinen Formel 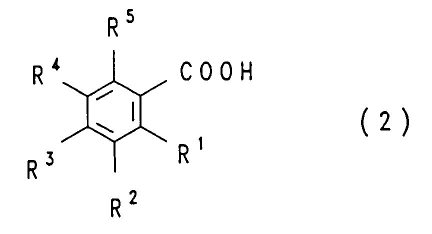 worin die Reste R¹, R², R³, R⁴ und R⁵ gleich oder verschieden sind und die vorstehend genannte Bedeutung haben oder abweichend hiervon anstelle von H für COOH stehen können, in Wasser löst,die wäßrige Lösung mit einem in Wasser nicht löslichen, unter den Reaktionsbedingungen inerten Amin versetzt und die Decarboxylierung in Anwesenheit oder Abwesenheit eines in Wasser nichtlöslichen, unter den Reaktionsbedingungen inerten Lösungsmittels und in Anwesenheit oder Abwesenheit eines Decarboxylierungskatalysators bei einem pH-Wert von 3 bis 9 und einer Temperatur von 70 bis 210°C durchführt.

2. Verfahren nach Anspruch 1, dadurch gekennzeichnet, daß man eine Verbindung der Formel (2) einsetzt, worin R¹, R², R³, R⁴ und R⁵ gleich oder verschieden sind und für COOH, H, F, Cl, OH oder einen Alkoxyrest mit 1 bis 4 Kohlenstoffatomen stehen.

3. Verfahren nach Anspruch 1 oder 2, dadurch gekennzeichnet, daß man eine Verbindung der Formel (2) einsetzt, worin R¹, R², R³, R⁴ und R⁵ gleich oder verschieden sind und für COOH, H, F, OH oder einen Alkoxyrest mit 1 bis 4 Kohlenstoffatomen stehen.

4. Verfahren nach einem oder mehreren der Ansprüche 1 bis 3, dadurch gekennzeichnet, daß man eine Verbindung der Formel (2) einsetzt, worin 0 bis 2 der Reste R¹ bis R⁵ für COOH stehen.

5. Verfahren nach einem oder mehreren der Ansprüche 1 bis 4, dadurch gekennzeichnet, daß man eine Verbindung der Formel (2) einsetzt, worin R¹ oder R⁵ für COOH stehen.

6. Verfahren nach einem oder mehreren der Ansprüche 1 bis 5, dadurch gekennzeichnet, daß man eine Verbindung der Formel (2) einsetzt, worin ein bis vier der Reste R¹ bis R⁵ für F stehen.

7. Verfahren nach einem oder mehreren der Ansprüche 1 bis 6, dadurch gekennzeichnet, daß man eine Verbindung der Formel (2) einsetzt, worin einer der Reste R¹ bis R⁵ für OH oder eine Alkoxygruppe mit 1 bis 4 Kohlenstoffatomen steht.

8. Verfahren nach einem oder mehreren der Ansprüche 1 bis 7, dadurch gekennzeichnet, daß man eine wäßrige Lösung einsetzt, die 1 bis 50, insbesondere 10 bis 30 Gew.-% der Verbindung der Formel (2) und gegebenenfalls weitere wasserlösliche Salze enthält.

9. Verfahren nach einem oder mehreren der Ansprüche 1 bis 8, dadurch gekennzeichnet, daß man je Mol der Verbindung der Formel (2) 0,001 bis 50, insbesondere 0,01 bis 2, bevorzugt 0,05 bis 1, besonders bevorzugt 0,1 bis 0,5 Mol des in Wasser nichtlöslichen Amins einsetzt.

10. Verfahren nach einem oder mehreren der Ansprüche 1 bis 9, dadurch gekennzeichnet, daß man als in Wasser nichtlösliches Amin ein Alkylamin mit 6 bis 30 Kohlenstoffatomen, ein Dialkylamin mit 6 bis 30 Kohlenstoffatomen je Alkylrest, ein Trialkylamin mit 4 bis 30 Kohlenstoffatomen je Alkylrest, eine N-enthaltende heterocyclische Verbindung oder ein Gemisch der vorstehend genannten Amine einsetzt.

11. Verfahren nach einem oder mehreren der Ansprüche 1 bis 10, dadurch gekennzeichnet, daß man als in Wasser nichtlösliches Amin ein Alkylamin mit 8 bis 20 Kohlenstoffatomen im Alkylrest, ein Dialkylamin mit 8 bis 20 Kohlenstoffatomen je Alkylrest, ein Trialkylamin mit 6 bis 20 Kohlenstoffatomen je Alkylrest, ein gegebenenfalls alkyliertes Chinolin oder Pyridin oder ein Gemisch der vorstehend genannten Amine einsetzt.

12. Verfahren nach einem oder mehreren der Ansprüche 1 bis 11, dadurch gekennzeichnet, daß man als in Wasser nichtlösliches Amin ein Trialkylamin mit 6 bis 20, insbesondere 6 bis 14 Kohlenstoffatomen je Alkylrest oder ein Gemisch dieser Trialkylamine einsetzt.

13. Verfahren nach einem oder mehreren der Ansprüche 1 bis 12, dadurch gekennzeichnet, daß man das inerte Lösungsmittel in einer Menge von 1 bis 200, insbesondere 2 bis 50, bevorzugt 5 bis 20 Vol.-%, bezogen auf die wäßrige Lösung einsetzt.

14. Verfahren nach einem oder mehreren der Ansprüche 1 bis 13, dadurch gekennzeichnet, daß man als inertes Lösungsmittel halogenierte oder nichthalogenierte aliphatische Kohlenwasserstoffe, halogenierte oder nichthalogenierte aromatische Kohlenwasserstoffe oder Ether, insbesondere chlorierte oder nicht chlorierte aromatische Kohlenwasserstoffe oder Ether oder ein Gemisch dieser Lösungsmittel einsetzt.

15. Verfahren nach einem oder mehreren der Ansprüche 1 bis 14, dadurch gekennzeichnet, daß man als Decarboxylierungskatalysator Kupfer, eine Kupfer(I)verbindung, eine Kupfer(II)verbindung, beispielsweise Kupfer(I)oxid, Kupfer(II)oxid, Kupfer(I)sulfat, Kupfer(II)sulfat, Kupfer(I)chlorid, Kupfer(II)chlorid, Kupfer(I)fluorid, Kupfer(II)fluorid, Kupfercarbonat, Kupfer(I)hydroxid oder Kupfer(II)hydroxid einsetzt.

16. Verfahren nach einem oder mehreren der Ansprüche 1 bis 15, dadurch gekennzeichnet, daß man 0,1 bis 10, insbesondere 0,5 bis 3 Mol% Decarboxylierungskatalysator, bezogen auf die Verbindung der Formel (2), einsetzt.

17. Verfahren nach einem oder mehreren der Ansprüche 1 bis 16, dadurch gekennzeichnet, daß man die Decarboxylierung bei einem pH-Wert von 3,5 bis 8, insbesondere 4 bis 7, durchführt.

18. Verfahren nach einem oder mehreren der Ansprüche 1 bis 17, dadurch gekennzeichnet, daß man die Decarboxylierung bei einer Temperatur von 80 bis 180, insbesondere 90 bis 160°C, durchführt.

19. Verfahren nach einem oder mehreren der Ansprüche 1 bis 17, dadurch gekennzeichnet, daß man die Decarboxylierung einer Verbindung der Formel (2), worin ein oder zwei der Reste R¹ bis R⁵ oder R¹ oder R⁵ für COOH stehen, zu einer Verbindung der Formel (1), worin ein oder zwei der Reste R¹ bis R⁵ oder R¹ oder R⁵ für COOH stehen, bei einer Temperatur von 80 bis 130, insbesondere 85 bis 125, bevorzugt 90 bis 120°C, durchführt.

## Claims

1. A process for the preparation of compounds of the formula 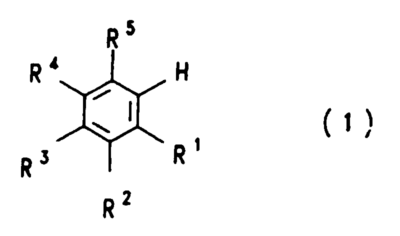 in which the radicals R¹, R², R³, R⁴ and R⁵ are identical or different and are COOH, H, F, Cl, Br, CF₃, OH, an alkoxy or alkyl radical having in each case 1 to 4 carbon atoms or a radical -NR⁶R⁷, in which R⁶ and R⁷ are identical or different and are H, an alkyl radical having 1 to 4 carbon atoms or a phenyl radical, which comprises dissolving in water a compound of the formula 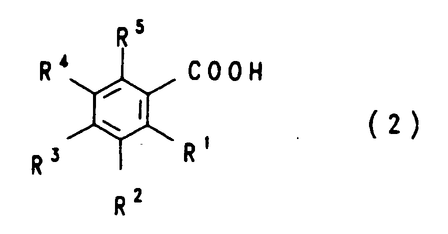 in which the radicals R¹, R², R³, R⁴ and R⁵ are identical or different and have the abovementioned meaning or, in departure therefrom, instead of H can be COOH, admixing the aqueous solution with a water-insoluble amine which is inert under the reaction conditions and carrying out the decarboxylation at a pH of 3 to 9 and a temperature of 70 to 210°C in the presence or absence of a water-insoluble solvent which is inert under the reaction conditions and in the presence or absence of a decarboxylation catalyst.

2. The process as claimed in claim 1, wherein a compound of the formula (2) is used, in which R¹, R², R³, R⁴ and R⁵ are identical or different and are COOH, H, F, Cl, OH or an alkoxy radical having 1 to 4 carbon atoms.

3. The process as claimed in claim 1 or 2, wherein a compound of the formula (2) is used, in which R¹, R², R³, R⁴ and R⁵ are identical or different and are COOH, H, F, OH or an alkoxy radical having 1 to 4 carbon atoms.

4. The process as claimed in one or more of claims 1 to 3, wherein a compound of the formula (2) is used, in which 0 to 2 of the radicals R¹ to R⁵ are COOH.

5. The process as claimed in one or more of claims 1 to 4, wherein a compound of the formula (2) is used, in which R¹ or R⁵ is COOH.

6. The process as claimed in one or more of claims 1 to 5, wherein a compound of the formula (2) is used, in which one to four of the radicals R¹ to R⁵ are F.

7. The process as claimed in one or more of claims 1 to 6, wherein a compound of the formula (2) is used, in which one of the radicals R¹ to R⁵ is OH or an alkoxy group having 1 to 4 carbon atoms.

8. The process as claimed in one or more of claims 1 to 7, wherein an aqueous solution is used which contains 1 to 50, in particular 10 to 30, % by weight of the compound of the formula (2) and, possibly, other water-soluble salts.

9. The process as claimed in one or more of claims 1 to 8, wherein 0.001 to 50, in particular 0.01 to 2, preferably 0.05 to 1, particularly preferably 0.1 to 0.5, mol of the water-insoluble amine are used per mole of the compound of the formula (2).

10. The process as claimed in one or more of claims 1 to 9, wherein the water-insoluble amine used is an alkylamine having 6 to 30 carbon atoms, a dialkylamine having 6 to 30 carbon atoms per alkyl radical, a trialkylamine having 4 to 30 carbon atoms per alkyl radical, an N-containing heterocyclic compound or a mixture of the abovementioned amines.

11. The process as claimed in one or more of claims 1 to 10, wherein the water-insoluble amine used is an alkylamine having 8 to 20 carbon atoms in the alkyl radical, a dialkylamine having 8 to 20 carbon atoms per alkyl radical, a trialkylamine having 6 to 20 carbon atoms per alkyl radical, an optionally alkylated quinoline or pyridine or a mixture of the abovementioned amines.

12. The process as claimed in one or more of claims 1 to 11, wherein the water-insoluble amine used is a trialkylamine having 6 to 20, in particular 6 to 14, carbon atoms per alkyl radical or a mixture of these trialkylamines.

13. The process as claimed in one or more of claims 1 to 12, wherein the inert solvent is used in an amount of 1 to 200, in particular 2 to 50, preferably 5 to 20, % by volume, based on the aqueous solution.

14. The process as claimed in one or more of claims 1 to 13, wherein, as inert solvent, use is made of halogenated or nonhalogenated aliphatic hydrocarbons, halogenated or nonhalogenated aromatic hydrocarbons or ethers, in particular chlorinated or nonchlorinated aromatic hydrocarbons or ethers or a mixture of these solvents.

15. The process as claimed in one or more of claims 1 to 14, wherein the decarboxylation catalyst used is copper, a copper(I) compound, a copper(II) compound, for example copper(I) oxide, copper(II) oxide, copper(I) sulfate, copper(II) sulfate, copper(I) chloride, copper(II) chloride, copper(I) fluoride, copper(II) fluoride, copper carbonate, copper(I) hydroxide or copper(II) hydroxide.

16. The process as claimed in one or more of claims 1 to 15, wherein 0.1 to 10, in particular 0.5 to 3, mol% of decarboxylation catalyst are used, based on the compound of the formula (2).

17. The process as claimed in one or more of claims 1 to 16, wherein the decarboxylation is carried out at a pH of 3.5 to 8, in particular 4 to 7.

18. The process as claimed in one or more of claims 1 to 17, wherein the decarboxylation is carried out at a temperature of 80 to 180, in particular 90 to 160, °C.

19. The process as claimed in one or more of claims 1 to 17, wherein the decarboxylation of a compound of the formula (2), in which one or two of the radicals R¹ to R⁵ or R¹ or R⁵ are COOH, to give a compound of the formula (1), in which one or two of the radicals R¹ to R⁵ or R¹ or R⁵ are COOH, is carried out at a temperature of 80 to 130, in particular 85 to 125, preferably 90 to 120, °C.

## Revendications

1. Procédé pour la préparation de composés de formule générale 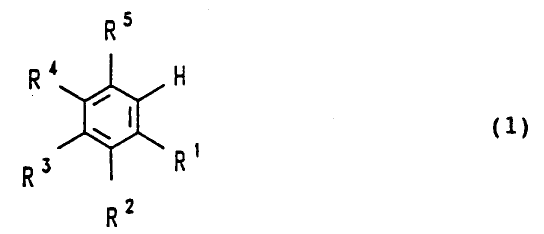 dans laquelle les restes R¹, R², R³, R⁴ et R⁵ sont identiques ou différents et représentent les groupes COOH, H, F, Cl, Br, CF₃, OH, un reste alkoxy ou alkyle chacun avec 1 à 4 atomes de carbone ou un reste -NR⁶R⁷, dans lequel R⁶ et R⁷ sont identiques ou différents et représentent un atome d'hydrogène, un reste alkyle avec 1 à 4 atomes de carbone ou un reste phényle. Il est caractérisé en ce que l'on dissout dans l'eau un composé de formule générale 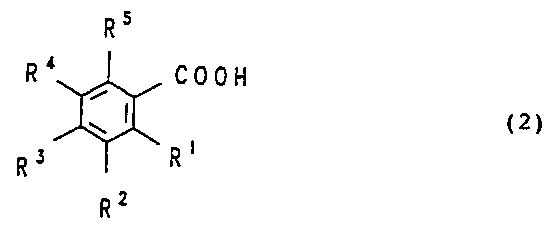 dans laquelle les restes R¹, R², R³, R⁴ et R⁵ sont identiques ou différents et possèdent la même signification que ci-dessus ou à la différence de celle-ci, peuvent représenter le groupe COOH au lieu d'un atome d'hydrogène, on ajoute à la solution aqueuse une amine insoluble dans l'eau, inerte dans les conditions réactionnelles, et on met en oeuvre la décarboxylation en présence ou en l'absence d'un solvant inerte dans les conditions réactionnelles et en présence ou en l'absence d'un catalyseur de décarboxylation, à une valeur de pH de 3 à 9 et une température de 70 à 210 °C.

2. Procédé selon la revendication 1, caractérisé en ce que l'on emploie un composé de formule (2), où R¹, R², R³, R⁴ et R⁵ sont identiques ou différents et représentent les groupes COOH, H, F, Cl, OH ou un reste alkoxy avec 1 à 4 atomes de carbone.

3. Procédé selon la revendication 1 ou 2, caractérisé en ce que l'on emploie un composé de formule (2), où R¹, R², R³, R⁴ et R⁵ sont identiques ou différents et représentent les groupes COOH, H, F, OH ou un reste alkoxy avec 1 à 4 atomes de carbone.

4. Procédé selon une ou plusieurs des revendications 1 à 3, caractérisé en ce que l'on emploie un composé de formule (2), où 0 à 2 des restes R¹ à R⁵ représentent le groupe COOH.

5. Procédé selon une ou plusieurs des revendications 1 à 4, caractérisé en ce que l'on emploie un composé de formule (2), où R¹ ou R⁵ représentent le groupe COOH.

6. Procédé selon une ou plusieurs des revendications 1 à 5, caractérisé en ce que l'on emploie un composé de formule (2), où un à quatre des restes R¹ à R⁵ représentent des atomes de F.

7. Procédé selon une ou plusieurs des revendications 1 à 6, caractérisé en ce que l'on emploie un composé de formule (2), où l'un des restes R¹ à R⁵ représente le groupe OH ou un groupe alkoxy avec 1 à 4 atomes de carbone.

8. Procédé selon une ou plusieurs des revendications 1 à 7, caractérisé en ce que l'on emploie une solution aqueuse contenant de 1 à 50, en particulier de 10 à 30 % en poids de composé de formule (2) et éventuellement un autre sel soluble.

9. Procédé selon une ou plusieurs des revendications 1 à 8, caractérisé en ce que l'on emploie, par mole de composé de formule (2), de 0,001 à 50, en particulier de 0,01 à, de préférence de 0,05 à 1, de manière particulièrement préférée de 0,1 à 0,5 mole de l'amine insoluble dans l'eau.

10. Procédé selon une ou plusieurs des revendications 1 à 9, caractérisé en ce que l'on emploie en tant qu'amine insoluble dans l'eau une alkylamine comportant de 6 à 30 atomes de carbone, une dialkylamine comportant de 6 à 30 atomes de carbone par reste alkyle, une trialkylamine comportant de 4 à 30 atomes de carbone par reste alkyle, un composé hétérocyclique contenant de l'azote ou un mélange des amines précitées.

11. Procédé selon une ou plusieurs des revendications 1 à 10, caractérisé en ce que l'on emploie en tant qu'amine insoluble dans l'eau une alkylamine comportant de 8 à 20 atomes de carbone dans le reste alkyle, une dialkylamine comportant de 8 à 20 atomes de carbone par reste alkyle, une trialkylamine comportant de 6 à 20 atomes de carbone par reste alkyle, une quinoléine ou pyridine éventuellement alkylée ou un mélange des amines précitées.

12. Procédé selon une ou plusieurs des revendications 1 à 11, caractérisé en ce que l'on emploie en tant qu'amine insoluble dans l'eau une trialkylamine comportant de 6 à 20 atomes de carbone, en particulier de 6 à 14 atomes de carbone par reste alkyle ou un mélange de ces trialkylamines.

13. Procédé selon une ou plusieurs des revendications 1 à 12, caractérisé en ce que l'on utilise la solvant inerte dans une quantité de 1 à 200, en particulier de 20 à 50, de préférence de 5 à 20 % en volume, par rapport à la solution aqueuse.

14. Procédé selon une ou plusieurs des revendications 1 à 13, caractérisé en ce que l'on emploie en tant que solvant inerte des hydrocarbures aliphatiques halogénés ou non halogénés, des hydrocarbures aromatiques halogénés ou non halogénés ou des éthers, en particulier des éthers ou des hydrocarbures aromatiques ou chlorés ou non chlorés ou un mélange de ces solvants.

15. Procédé selon une ou plusieurs des revendications 1 à 14, caractérisé en ce que l'on emploie en tant que catalyseur de décarboxylation le cuivre, un composé de cuivre(I), un composé de cuivre(II), par exemple l'oxyde de cuivre(I), l'oxyde de cuivre(II), le sulfate de cuivre(I), le sulfate de cuivre(II), le chlorure de cuivre(I), le chlorure de cuivre(II), le fluorure de cuivre(I), le fluorure de cuivre(II), le carbonate de cuivre, l'hydroxyde de cuivre(I), l'hydroxyde de cuivre(II).

16. Procédé selon une ou plusieurs des revendications 1 à 15, caractérisé en ce que l'on emploie de 0,1 à 10, en particulier de 0,5 à 3 % en moles de catalyseur de décarboxylation, par rapport au composé de formule (2).

17. Procédé selon une ou plusieurs des revendications 1 à 16, caractérisé en ce que l'on réalise la décarboxylation à une valeur de pH de 3,5 à 8, en particulier de 4 à 7.

18. Procédé selon une ou plusieurs des revendications 1 à 17, caractérisé en ce que l'on réalise la décarboxylation à une température de 80 à 180, en particulier de 90 à 160 °C.

19. Procédé selon une ou plusieurs des revendications 1 à 17, caractérisé en ce que l'on réalise la décarboxylation d'un composé de formule (2), où un ou deux des restes R¹ à R⁵ ou R¹ ou R⁵ représentent le groupe COOH, pour obtenir un composé de formule (1), où un ou deux des restes R¹ à R⁵ ou R¹ ou R⁵ représentent le groupe COOH, à une température de 80 à 130, en particulier de 85 à 125, de préférence de 90 à 120 °C.
